# EUROPÄISCHE PATENTANMELDUNG

(11) **EP 2 708 528 A1**
(43) Veröffentlichungstag der Anmeldung: **19.03.2014**
(21) Anmeldenummer: 13180277.9
(22) Anmeldetag: 13.08.2013
(51) Int. Cl.: C07C 67/29, C07C 69/54, C07C 303/28, C07C 309/73

(54) **Photoreaktive Monomere**

(30) Priorität: 12.09.2012 DE 102012216171
(71) Anmelder: BASF SE, 67056 Ludwigshafen (DE); Karlsruher Institut Für Technologie (KIT), 76131 Karlsruhe (DE)
(72) Erfinder: Urban, Dieter, 67346 Speyer (DE); Licht, Ulrike, 68309 Mannheim (DE); Barner-Kowollik, Christopher, 76297 Stutensee-Blankenloch (DE); Delaittre, Guillaume, 68165 Mannheim (DE); Frick, Elena, 76137 Karlsruhe (DE)

(57) **Zusammenfassung**

Photoreaktives Monomer, das (i) mindestens eine radikalisch polymerisierbare C-C-Doppelbindung, (ii) mindestens eine hydrophile Gruppe, ausgewählt aus einer Ethylenglykolgruppe und einer Polyethylenglykolgruppe mit mindestens 2 Ethylenglykoleinheiten und (iii) mindestens eine photoreaktive Gruppe aufweisen, wobei die photoreaktive Gruppe eine photoenolisierbare Carbonylgruppe ist, sowie ein Verfahren zur Herstellung der photoreaktiven Monomere.

## Beschreibung

Die vorliegende Erfindung betrifft ein photoreaktives Monomer, das mindestens eine radikalisch polymerisierbare C-C-Doppelbindung, mindestens eine bestimmte hydrophile Gruppe und mindestens eine photoreaktive Gruppe aufweist, sowie ein Verfahren zur Herstellung des photoreaktiven Monomeren.

Photoreaktive Monomere werden zur Herstellung von wässrigen Polymerdispersionen eingesetzt, aus denen Polymerfilme gebildet werden. Derartige Filme müssen oft zur Erreichung der notwendigen und gewünschten anwendungstechnischen Eigenschaften so vernetzt werden, dass eine interpartikuläre Vernetzungsreaktion zwischen den Polymerpartikeln stattfindet. Dazu werden photoreaktive Monomere eingesetzt, die in einer Emulsionspolymerisation so umgesetzt werden, dass sie an der Partikeloberfläche sitzen. Die Filmbildung erfolgt dann in der Regel durch gleichmäßiges Austrocknen bei Raumtemperatur an Luft. Anschließend erfolgt die Vernetzung durch Lichteinwirkung.

Grundsätzlich sind funktionelle Spezialmonomer zur Vernetzung von wässrigen Polymerdispersionen oder Polymeren bekannt. Diese bieten neben der polymerisationsfähigen Gruppe noch weitere chemische Gruppierungen an, die miteinander oder mit anderen Gruppen reagieren können. Die meisten bekannten Monomere sind wegen ihrer schlechten Wasserlöslichkeit aber entweder schlecht in wässrigen Emulsionspolymerisaten oder Lösungen einsetzbar, oder ihre weitere funktionelle Gruppe benötigt hohe Temperaturen, um zur Reaktion zu kommen.

Demgemäß war es die Aufgabe der vorliegenden Erfindung neue funktionelle Spezialmonomere zu finden, die eine steuerbare Reaktivität möglichst bei Raumtemperatur und gute Wasserlöslichkeit bieten.

Gelöst wurde diese Aufgabe durch photoreaktive Monomere, die
(i) mindestens eine radikalisch polymerisierbare C-C-Doppelbindung,
(ii) mindestens eine hydrophile Gruppe, ausgewählt aus einer Ethylenglykolgruppe und Polyethylenglykolgruppe mit mindestens 2 Ethylenglykoleinheiten und
(iii) mindestens eine photoreaktive Gruppe aufweisen, wobei die photoreaktive Gruppe eine photoenolisierbare Carbonylgruppe ist.

Gegenstand der Erfindung ist auch ein Verfahren zur Herstellung photoreaktiver Monomere.

Im Folgenden wird die Bezeichnung (Meth)acrylat und ähnliche Bezeichnungen als abkürzende Schreibweise verwendet für "Acrylat oder Methacrylat".

Der Begriff "photoreaktiv" betrifft Verbindungen, die photoinduzierte chemische Reaktionen eingehen können, d.h. deren chemische Reaktivität durch Lichteinwirkung erhöht wird.

Die photoreaktiven Monomere weisen mindestens eine, vorzugsweise eine einzige radikalisch polymerisierbare C-C-Doppelbindung auf. Besonders geeignet sind z.B. Monomere, bei denen die radikalisch polymerisierbare C-C-Doppelbindung Teil einer Acrylat- oder Methacrylatgruppe ist.

Die photoreaktiven Monomere weisen außerdem mindestens eine hydrophile Gruppe auf. Die hydrophile Gruppe bewirkt, dass sich in Polymerdispersionen die räumlich benachbarte photoreaktive Gruppe zusammen mit der hydrophilen Gruppe an der Oberfläche von dispergierten Polymerpartikeln befindet und so für photoinduzierte Reaktionen mit Verbindungen, die sich in der wässrigen Phase befinden oder die auf der Oberfläche anderer Polymerpartikel angesiedelt sind, zur Verfügung stehen.

Bei der hydrophilen Gruppe handelt es sich um eine Gruppe mit einer oder mehreren Ethylenglykolgruppen, z.B. 2 bis 30, vorzugsweise 3 bis 30 oder 3 bis 20, insbesondere 4 bis 20 oder 4 bis 10 Ethylenglykolgruppen. Weist das übrige Monomer höhere Anteile an hydrophoben Strukturelementen auf, werden vorzugsweise mehr Ethylenglykolgruppen verwendet, z.B. mindestens 5 bis 20 oder mehr. Weist das übrige Monomer nur geringe Anteile an hydrophoben Strukturelementen auf, werden vorzugsweise weniger Ethylenglykolgruppen verwendet, z.B. 1 bis 20 oder 2 bis 10.

Die photoreaktiven Monomere weisen außerdem mindestens eine photoreaktive Gruppe auf, wobei die photoreaktive Gruppe vorzugsweise eine photoenolisierbare Carbonylgruppe ist. Eine photoenolisierbare Carbonylgruppe ist eine Keto- oder Aldehydgruppe, welche unter Lichteinwirkung in das jeweilige Tautomere Enol umgewandelt werden kann. Derartige Verbindungen werden im Folgenden auch abkürzend als "Photoenole" bezeichnet.

Bevorzugte photoenolisierbare Carbonylgruppen sind photoenolisierbare alpha-Aryl-Carbonylgruppen. Geeignet sind z.B. Verbindungen, bei denen die photoreaktive Gruppe abgeleitet ist von einer Struktureinheit der Formel 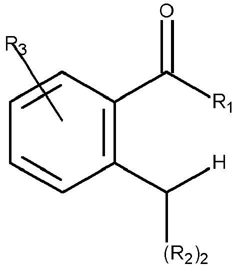 wobei R₁ für Wasserstoff oder einen organischen Rest steht, z.B. eine Alkyl- oder Arylgruppe, R₂ für Wasserstoff oder einen organischen Rest steht, z.B. eine Alkylgruppe, vorzugsweise Methyl und R₃ für die Substituenten des aromatischen Ringes steht, welche gleich oder voneinander verschieden sind und über eine oder mehrere Ringstrukturen miteinander verbunden sein können, z.B. Wasserstoff, Alkyl- oder Arylgruppen.

Unter einer Alkylgruppe im Sinne der Erfindung wird bevorzugt ein C₁-C₂₀-Alkylrest verstanden. Dies sind geradkettige oder verzweigte Kohlenwasserstoffreste mit bis zu 20 Kohlenstoffatomen, bevorzugt C₁-C₁₀-Alkyl wie Methyl, Ethyl, Propyl, Isopropyl, n-Butyl, sec-Butyl, tert.-Butyl, 1,1-Dimethylethyl, Pentyl, 2-Methylbutyl, 1,1-Dimethylpropyl, 1,2-Dimethylpropyl, 2,2-Dimethylpropyl, 1-Ethylpropyl, Hexyl, 2-Methylpentyl, 3-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Heptyl, Octyl, 2-Ethylhexyl, 2,4,4-Trimethylpentyl, 1,1,3,3-Tetramethylbutyl, Nonyl und Decyl sowie deren Isomere.

Eine Arylgruppe im Sinne der Erfindung ist ein ein- bis dreikerniges aromatisches Ringsystem enthaltend 6 bis 14 Kohlenstoffringglieder, z. B. Phenyl, Naphthyl und Anthracenyl, bevorzugt ein ein- bis zweikerniges, besonders bevorzugt ein einkerniges aromatisches Ringsystem.

Bevorzugte photoreaktive Monomere sind Verbindungen mit der Struktur der Formel (I) 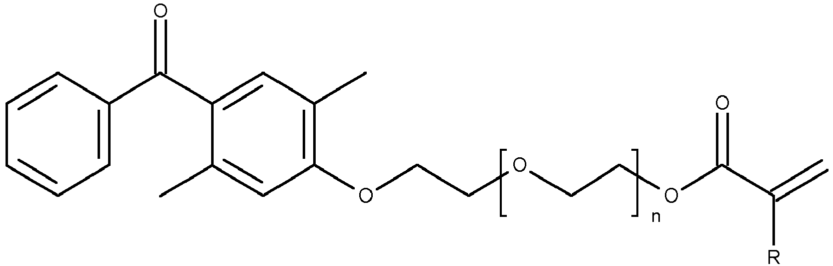 wobei n eine Zahl von 0 bis 29, vorzugsweise von 3 bis 19 bedeutet und R Wasserstoff oder Methyl bedeutet.

Bevorzugte photoreaktive Monomere sind auch Verbindungen mit der Struktur der Formel (II) 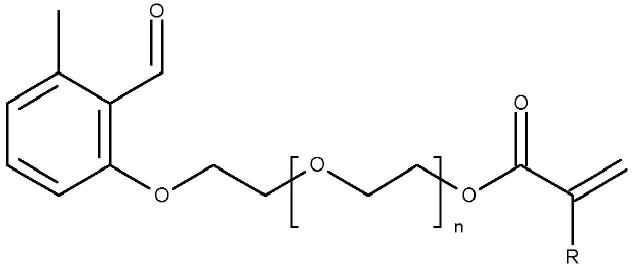 wobei n eine Zahl von 0 bis 19, vorzugsweise 1 bis 9 bedeutet und R Wasserstoff oder Methyl bedeutet.

Gegenstand der Erfindung ist ebenfalls ein Verfahren zur Herstellung der photoreaktiven Verbindungen.

Grundsätzlich ist die katalytische Ethoxylierung photoreaktiver Gruppen wie 4-Hydroxy-2,5-dimethyl-phenyl-phenylmethanon möglich, läuft aber mit schlechten Ausbeuten, da die Reaktivität des Eduktes weit unter der der ethoxylierten Stufe liegt.

Daher werden Verbindungen der Formel (I) bevorzugt ausgehend von einem Ethylenglykol mit der entsprechenden Anzahl an Ethylenglykolgruppen n = 0 bis 29 mit p-Toluolsulfonylchlorid umgesetzt. Anschließend erfolgt die Umsetzung mit einer Verbindung, die eine photoreaktive Gruppe enthält, wobei diese photoreaktive Gruppe wie oben beschrieben eine photoenolisierbare Carbonylgruppe ist. In der Folgereaktion erfolgt die Einbringung der radikalisch polymerisierbaren C-C-Doppelbindung durch Umsetzung mit einem (Meth)acryloylhalogenid, bevorzugt einem (Meth)acryloylchlorid. Alternativ kann die letzte Stufe auch die saure Veresterung mit (Meth)acrylsäure nach den bekannten Methoden erfolgen, wobei sich Katalysatoren wie beispielsweise saure Ionenaustauscher besser noch p-Toluolsulfonsäure, Methansulfonsäure und H₂SO₄ eignen. Alternativ dazu eignen sich auch enzymatische Katalysatoren wie beispielsweise eine Lipase aus *Candida Antarctica B,* die im Handel unter dem Namen Novozym 435 erhältlich sind. Gut geeignet ist auch die Umesterung von (Meth)acrylsäureestern mittels Lewis-Säuren, wie Dibutylzinndioxid oder Titan- tetra-isopropoxylat, oder mit anorganischen Salzen wie Alkali-oder Erdalkalicarbonate, -hydrogencarbonate, -phosphate, -hydrogenphosphate, - dihydrogenphosphate, -sulfate oder -sulfite. Bevorzugte anorganische Salze für eine Umesterungsreaktion sind Li₃PO₄, K₃PO₄, Na₃PO₄, K₂CO₃ und Na₂CO₃.

Verbindungen der Formel (II) werden bevorzugt ausgehend von einem Poly(ethylenglykol)(meth)acrylat hergestellt, d.h. diese Ausgangsverbindung enthält bereits die hydrohile Gruppe sowie die radikalisch polymerisierbare C-C-Doppelbindung. Anschließend wird dieses Edukt zur Einbringung der photoreaktiven Gruppe zunächst mit p-Toluolsulfonylchlorid umgesetzt und anschließend mit einer Verbindung, die eine photoreaktive Gruppe enthält, wobei diese photoreaktive Gruppe wie oben beschrieben eine photoenolisierbare Carbonylgruppe ist.

Die Synthesen der Verbindungen der Formeln (I) und (II) sind prinzipiell aber austauschbar.

Die photoreaktiven Gruppen, die den Verbindungen der Formel (I) zugrunde liegen, können wie in der Literatur beschrieben über eine Fries-Umlagerung aus einfachen Ausgangskomponenten dargestellt werden (C. Barner-Kowollik, Macromol .Rapid Commun. 32 (11), 807 2011 (supporting information)). Eine Herstellungsmöglichkeit über katalytische Prozesse findet sich in DE 69321830. Die Herstellung der photoreaktiven Gruppen der Verbindungen der Formel (II) sind in C. Barner-Kowollik, Angew. Chem Int. Ed. 51,1071, 2012, supporting information, beschrieben.

Die photoreaktiven Monomere können zur Herstellung von wässrigen Polymersdispersionen bzw. Lösungen eingesetzt werden, die im Bereich der filmbildenden Produkte (Klebstoffe, Beschichtungen, Farben und Lacke) verwendet werden. Diese Polymerisate können die Stabilität der Filme erhöhen durch die Anwendung von Licht, es wird keine hohe Temperatur benötigt. Sie besitzen Einsatzmöglichkeiten in wässrigen Emulsionspolymerisaten verschiedener Anwendungen zur Modifizierung der Teilchenoberflächen oder der daraus gebildeten Filme. Produkte wären z.B. Klebstoffe, deren Klebekraft durch Bestrahlung mit Licht verstärkt wird. Weitere Produkt wären Farben oder Lacke, die nach der Bestrahlung mit Licht eine höhere Abriebfestigkeit besitzen und wasserunlöslich sind.

### Beispiele

### Beispiel 1: Synthese von Photoenol-Monomer 1

### Syntheseschema:

### Synthese von Tos-PEG-OH (1):

Tetraethylenglykol (15 g, 77,2 mmol) werden in 220 ml Acetonitril gelöst. Triethylamine wird unter Stickstoffatmosphäre tropfenweise zugegeben. Anschließend wird p-Toluolsulfonylchlorid (14,718 g, 77,2 mmol), gelöst in 75 ml Acetonitril, tropfenweise bei 0°C zugegeben. Nach 12 h Rühren bei Raumtemperatur wird filtriert und das Lösungsmittel unter vermindertem Druck entfernt. Reinigung durch Flash-Chromatographie (Silicagel, Chloroform + 10% Methanol, Rf = 0,68) gibt ein gelbes Öl (Ausbeute: 31 %).

¹H-NMR (CDCl₃, 250 MHz):δ/ppm: 2,37 (s, 3H, CH₃), 2,68 (bs, 1H, OH), 3,50-3,65 (m, 14H, CH₂O), 4,09 (t, 2H, CH₂O), 7,29 (d, 2H, Hₐᵣ), 7,70 (d, 2H, Hₐᵣ).

### Synthese von PE-PEG-OH (2):

4-Hydroxy-2,5-dimethyl-phenyl-phenylmethanon (16,956 g, 74,9 mmol) wird in 140 ml wasserfreiem Aceton unter Stickstoffatmosphäre gelöst. Anschließend wird K₂CO₃ (10,352 g, 74,9 mmol) zugefügt. Eine Lösung von (1) (15,358 g, 44,1 mmol) in 40 ml wasserfreiem Aceton wird tropfenweise bei Raumtemperatur zugegeben. Die Mischung wird für 39 h bei 60°C unter Rückfluss erhitzt. Nach Abkühlen auf Raumtemperatur wird filtriert und das Lösungsmittel unter vermindertem Druck entfernt. Reinigung durch Flash-Chromatographie (Silicagel, Et₂O + 10% Methanol, Rf = 0,53) gibt ein leicht gelbes Öl (Ausbeute: 61 %).

¹H-NMR (CDCl₃, 250 MHz):δ/ppm: 2,10 (s, 3H, CH₃), 2,29 (s, 3H, CH₃), 2,42 (bs, 1H, OH), 3,51-3,71 (m, 12H, CH₂O), 3,83 (t, 2H, CH₂O), 4,13 (t, 2H, CH₂O), 6,65 (s, 1H, Hₐᵣ), 7,08 (s, 1H, Hₐᵣ), 7,33-7,40 (m, 2H, Hₐᵣ), 7,45-7,51 (m, 1H, Hₐᵣ), 7,67-7,71 (m 2H, Hₐᵣ).

### Synthese von PE-PEG-Acrylat (3):

Acryloylchlorid (1 ml, 12,4 mmol) wird in 10 ml wasserfreiem Dichlormethan unter Stickstoffatmosphäre gelöst. Eine Lösung von (2) (2 g, 4,97 mmol) und Triethylamin (2,08 ml, 14,9 mmol) in 15 ml wasserfreiem Dichlormethan werden tropfenweise bei 0°C zugegeben. Nach der Zugabe wird auf Raumtemperatur erwärmen gelassen und für 24 h gerührt. Die Lösung wird mit Wasser (2 x 25 ml) und Salzlösung (2 x 25 ml) gewaschen und über MgSO₄ getrocknet. Das Lösungsmittel wird unter vermindertem Druck entfernt. Reinigung durch Flash-Chromatographie (Silicagel, Et₂O + 5% Methanol, Rf = 0,80) gibt ein leicht gelbes Öl (Ausbeute: 72%).

¹H-NMR (CDCl₃, 250 MHz):δ/ppm: 2,10 (s, 3H, CH₃), 2,30 (s, 3H, CH₃), 3,61-3,72 (m, 10H, CH₂O), 3,84 (t, 2H, CH₂O), 4,13 (t, 2H, CH₂O), 4,25 (t, 2H, CH₂O), 5,76 (dd, 1H, CH), 6,08 (dd, 1H, CH), 6,36 (dd, 1H, CH), 6,64 (s, 1H, Hₐᵣ), 7,08 (s, 1H, Hₐᵣ), 7,35-7,41 (m, 2H, Hₐᵣ), 7,46-7,52 (m, 1H, Hₐᵣ), 7,67-7,71 (m 2H, Hₐᵣ).

### Beispiel 2: Synthese von Photoenol-Monomer 2

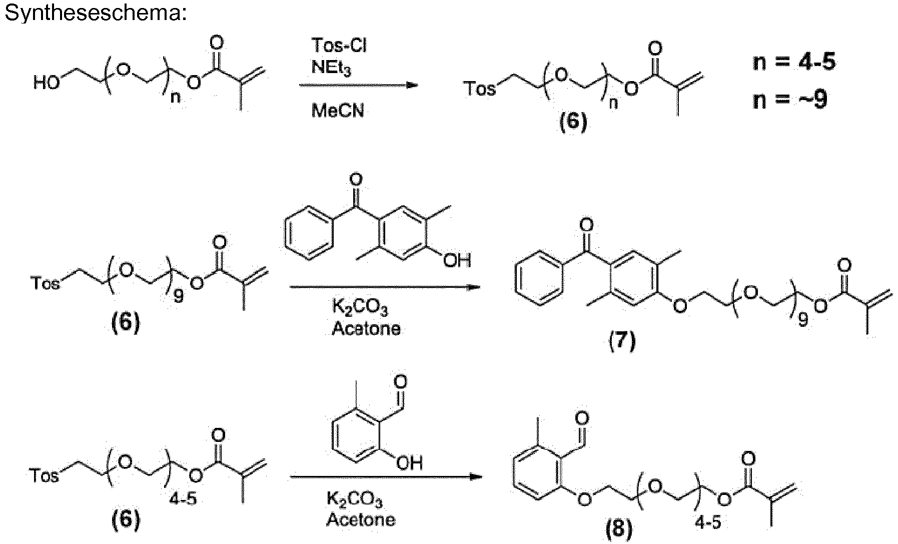

### Synthese von Verbindung (6):

Beschrieben wird die Synthese für n = 4-5; für n = 9 kann das gleiche Herstellverfahren angewendet werden.

Triethylamin (4,28 ml, 30,7 mmol) wird unter Stickstoffatmosphäre zu einer Lösung von Poly(ethylenglykol)methacrylat (Mn = 360, 11,05 g, 30,7 mmol) in 100 ml Acetonitril gegeben. p-Toluolsulfonylchlorid (5,852 g, 30,7 mmol) wird in 50 ml Acetonitril gelöst und tropfenweise bei 0 °C zugegeben. Nach der Zugabe wird auf Raumtemperatur erwärmen gelassen und für 12 h gerührt. Die Lösung wird filtriert und nach Zugabe von Hydrochinon (0,3 g) wird das Lösungsmittel unter vermindertem Druck bei 25 °C entfernt. Das Rohprodukt wird ohne weitere Reinigung weiterverwendet.

### Synthese von Verbindung (7):

4-Hydroxy-2,5-dimethyl-phenyl-phenylmethanon (0,7976 g, 3,53 mmol) wird in 15 ml wasserfreiem Aceton gelöst. Hydrochinon (0,1 g) und K₂CO₃ (0,69 g, 4,99 mmol) werden zugefügt. Nach tropfenweiser Zugabe einer Lösung von (6) (n = 9, 2,0 g, 2,94 mmol) in 25 ml wasserfreiem Aceton bei Raumtemperatur wird die Mischung für 22 h bei 60°C unter Rückfluss erhitzt. Das Lösungsmittel wird unter vermindertem Druck entfernt unter Bildung eines dunkelbraunen Öls.

### Synthese von Verbindung (8):

2-Hydroxy-6-methyl-benzaldehyd (Photoenol 2, 2,0 g, 14,7 mmol) wird in 40 ml wasserfreiem Aceton gelöst. Hydrochinon (0,1 g) und K₂CO₃ (3,385 g, 24,5 mmol) werden zugefügt. Nach tropfenweiser Zugabe einer Lösung von (6) (n = 4-5 6,273 g, 12,2 mmol) in 30 ml wasserfreiem Aceton bei Raumtemperatur wird die Mischung für 18 h bei 60°C unter Rückfluss erhitzt. Das Lösungsmittel wird unter vermindertem Druck entfernt unter Bildung eines dunkelbraunen Öls.

## Patentansprüche

1. Photoreaktives Monomer, das
(i) mindestens eine radikalisch polymerisierbare C-C-Doppelbindung,
(ii) mindestens eine hydrophile Gruppe, ausgewählt aus einer Ethylenglykolgruppe und einer Polyethylenglykolgruppe mit mindestens 2 Ethylenglykoleinheiten und
(iii) mindestens eine photoreaktive Gruppe aufweisen, wobei die photoreaktive Gruppe eine photoenolisierbare Carbonylgruppe ist.

2. Photoreaktives Monomer gemäß Anspruch 1, **dadurch gekennzeichnet, dass** die radikalisch polymerisierbare C-C-Doppelbindung Teil einer Acrylat- oder Methacrylatgruppe ist.

3. Photoreaktives Monomer gemäß Anspruch 1 oder 2, **dadurch gekennzeichnet, dass** die hydrophlie Gruppe eine Gruppe mit 2 bis 30 Ethylenglykolgruppen ist.

4. Photoreaktives Monomer gemäß einem der vorstehenden Ansprüche, **dadurch gekennzeichnet, dass** die photoreaktive Gruppe eine photoenolisierbare alpa-Aryl-Carbonylgruppe ist.

5. Photoreaktives Monomer gemäß einem der vorstehenden Ansprüche, **dadurch gekennzeichnet, dass** die photoreaktive Gruppe abgeleitet ist von einer Struktureinheit der Formel 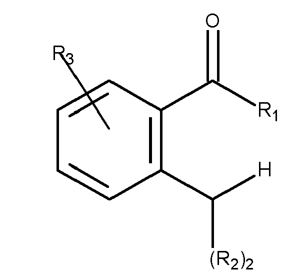 wobei R₁ für Wasserstoff oder einen organischen Rest steht, R₂ für Wasserstoff oder einen organischen Rest steht und R₃ für die Substituenten des aromatischen Ringes steht, welche gleich oder voneinander verschieden sind und über eine oder mehrere Ringstrukturen miteinander verbunden sein können.

6. Photoreakives Monomer gemäß einem der vorstehenden Ansprüche, **dadurch gekennzeichnet, dass** das photoreaktive Monomer ausgewählt ist aus Verbindungen mit der Struktur der Formel (I) 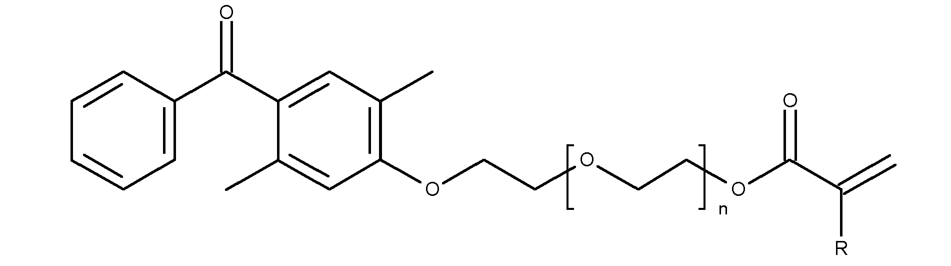 wobei n eine Zahl von 0 bis 29 bedeutet und R Wasserstoff oder Methyl bedeutet, oder Verbindungen mit der Struktur der Formel (II) 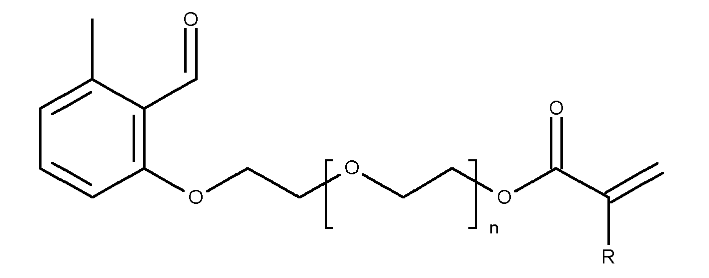 wobei n eine Zahl von 0 bis 19 und R Wasserstoff oder Methyl bedeutet.

7. Verfahren zur Herstellung einer Verbindung mit der Struktur der Formel (I) bestehend aus den Schritten
(I) Umsetzung eines Ethylenglykols mit der entsprechenden Anzahl an Ethylenglykolgruppen n = 0 bis 29 mit p-Toluolsulfonylchlorid,
(II) Umsetzung mit einer Verbindung, die eine photoreaktive Gruppe gemäß Anspruch 5 enthält, und
(III) Umsetzung mit einem (Meth)acryloylhalogenid.

8. Verfahren zur Herstellung einer Verbindung mit der Struktur der Formel (II) bestehend aus den Schritten
(I) Umsetzung eines Poly(ethylenglkyl)(meth)acrylats mit p-Toluolsulfonylchlorid
(II) Umsetzung mit einer Verbindung, die eine photoreaktive Gruppe gemäß Anspruch 5 enthält.
